# EUROPÄISCHE PATENTSCHRIFT

(11) **EP 1 956 963 B1**
(45) Veröffentlichungstag und Bekanntmachung des Hinweises auf die Patenterteilung: **19.11.2014**
(21) Anmeldenummer: 06846934.5
(22) Anmeldetag: 30.11.2006
(51) Int. Cl.: A61B 1/00, A61B 1/005, A61B 1/01

(54) **ANORDNUNG ZUR FÜHRUNG VON INSTRUMENTEN IN HOHLRÄUMEN**
ARRANGEMENT FOR GUIDING INSTRUMENTS IN CAVITIES
INSTALLATION DE GUIDAGE D'INSTRUMENTS DANS DES CAVITÉS

(30) Priorität: 30.11.2005 DE 102005057479
(43) Veröffentlichungstag der Anmeldung: 20.08.2008
(73) Patentinhaber: Philipps-Universität Marburg, 35037 Marburg (DE)
(72) Erfinder: MAISCH, Bernhard, 35043 Marburg (DE); RUPP, Heinz, 35043 Marburg (DE); RUPP, Thomas Philipp, 35043 Marburg (DE)
(74) Vertreter: Patentanwälte Olbricht Buchhold Keulertz
(86) Internationale Anmeldenummer: PCT/DE2006/002116
(87) Internationale Veröffentlichungsnummer: WO 2007/062632

(56) Entgegenhaltungen:
- EP-A- 1 547 514
- EP-A- 1 654 976
- WO-A-2006/123397
- US-A- 6 007 482

## Beschreibung

Die Erfindung betrifft eine Anordnung zur Führung von Instrumenten, insbesondere medizinischen Instrumenten, wie z.B. Endoskope, Entnahme- oder Montage-Werkzeuge oder auch Werkzeuge zur optischen Abbildung ausgesuchter Bereiche in Hohlräumen. Unter Hohlräumen werden solche in Lebewesen, aber auch in technischen Apparaten, wie z.B. Hohlräume in Turbinen verstanden.

Die Anordnung dient insbesondere der Führung flexibler Instrumente, so dass durch Abwinklung der Instrumente oder der Anordnung die Instrumente auch um Ecken herum geführt werden können.

Zur Führung und auch zur Ablenkung von Instrumenten, insbesondere medizinischen Instrumenten sind schon zahlreiche Vorrichtungen bekannt.

Dazu gehört z.B. EP 1 547 514, welches eine Endoskopvorrichtung mit aufblasbaren Ballons an der Führungshilfe offenbart, oder das in der DE 197 27 419 beschriebene Endoskop. Flexible Endoskope, oder solche, welche um eine Ecke herum geformt oder geführt werden können, sind ebenfalls bereits länger bekannt. Ein Beispiel dafür ist das in der DE 200 03 207 beschriebene Endoskop.
Aus der DE 201 05 206 ist auch ein zu dieser Gruppe gehöriges "Endoskopisches Intubationssystem" bekannt.

Für den eher technischen Bereich, sei das Beispiel einer Anordnung zur Führung flexibler Endoskope aus der DD 282 829 genannt.

Bei den vorgenannten Beispielen erfolgt die Abwinklung der Instrumente meist durch Drahtzüge.

### Nachteile im Stand der Technik

Alle vor bekannten Vorrichtungen weisen den Nachteil auf, dass eine Führung von Instrumenten mit den bekannten Vorrichtungen zwar sehr exakt innerhalb der Vorrichtungen möglich ist, jedoch die Führung relativ zu den Wandungen der Hohlräume in den meisten Fällen nur unzuverlässig möglich ist. Nur in den Fällen, in denen der Aussendurchmesser der Vorrichtungen, in welchen die Instrumente geführt werden zufällig dem Innendurchmesser der Hohlräume entspricht, ist eine exakte Führung der Instrumente auch relativ zu den Wänden des Hohlraumes möglich. Häufig wird daher eine einmal gefundene optimale Position nach dem Verschieben der Vorrichtungen oder auch der Instrumente nicht wieder aufgefunden, oder es kommt zu unkontrollierten Verschiebungen der Vorrichtungen oder der darin geführten Instrumente. Dienen die Instrumente, wie meist der Fall, zur Durchführung von Punktionen oder dem Einsetzen von Sonden oder Sensoren kann es durch diese Verschiebungen leicht zu falschen Positionierungen oder gar zu Verletzungen/Beschädigungen der Wandungen der Hohlräume kommen.

Nachteilig an den bekannten Vorrichtungen ist zusätzlich und auch, dass der Vorschub der Vorrichtungen z.B. in überwiegend kanalartigen Hohlräumen bis auf die zufällige Führung durch die Wandungen der Hohlräume meist unkontrolliert erfolgt.

### Aufgabe der Erfindung

Aufgabe der vorliegenden Erfindung ist es daher, eine Anordnung bereit zu stellen, welche die Voraussetzungen für eine zuverlässige Führung von Instrumenten relativ zu mindestens einem Teil der Wände eines Hohlraumes ermöglicht.

Im Falle von medizinischen oder tiermedizinischen Instrumenten ist erst durch eine solche Anordnung z.B. die definierte Untersuchung oder/und zuverlässige Punktion oder/und anderweitige Manipulation oder/und Sichtbarmachung von menschlichen bzw. tierischen Gefäßen oder Geweben möglich.

Die gestellte Aufgabe wird erfindungsgemäß durch eine Anordnung gemäß Anspruch 1 gelöst.

Um die bestehenden Probleme zu lösen, wurde eine erfindungsgemäße Anordnung entwickelt, welche eine Fixierung der Anordnung innerhalb des Hohlraumes durch der Anordnung zugeordnete Fixierungsmittel ermöglicht. Die Fixierungsmittel sorgen dabei für eine Fixierung der Anordnung relativ zu mindestens einem Teil der Wandung des zu untersuchenden Hohlraumes, in welche die Anordnung zumindest teilweise eingeführt sein muss.

Die Fixierungsmittel sind dabei so ausgeführt, dass diese bei Aktivierung mit zumindest einem Teil der Wandung des Hohlraumes in Kontakt, z.B. reibschlüssigen Kontakt treten und die Anordnung dadurch relativ zu diesem Wandungsteil fixieren. Die so fixierte Anordnung bietet für eine dann nachfolgende Bewegung des Instrumentes, welches z.B. innerhalb der Anordnung geführt wird, eine verbesserte Fixierung um zuverlässige Manipulationen oder optische Untersuchungen etc. innerhalb des Hohlraumes oder von dessen Wandungen ausgehend durchzuführen.

Zur Verbesserung der Führung bei Vor- oder Zurückbewegungen der Instrumente bietet eine erste Ausführungsform der erfindungsgemäßen Anordnung ebenfalls eine gegenüber dem Stand der Technik stark verbesserte Führung.
Die Anordnung weist dazu mindestens zwei von einander in Richtung der gewünschten Fortbewegung (z.B. vor oder zurück) zueinander beabstandete, an der Anordnung angebrachte Fixierungsmittel auf, welche zur Führung der Fortbewegung nacheinander aktivierbar ausgeführt sind.
Ferner weist die Anordnung dazu Fortbewegungsmittel auf. Diese sind so ausgeführt, dass nach Aktivierung der ersten Fixierungsmittel und Aktivierung der Fortbewegungsmittel ein nicht fixierter Teilbereich der Anordnung in Richtung der gewünschten Bewegung fortbewegt wird.

Mit Fixierungsmittel sind auch solche sich nach einer gewissen Dauer oder unter Einwirkung von chemischen oder physikalischen Einflüssen, wie durch Lösungsmittel oder durch elektromagnetische Strahlung oder Temperaturvariation auflösende Kleber oder andere chemische Substanzen gemeint. Diese werden von der Oberfläche der Anordnung in ausreichender Menge abgegeben und bilden nach einiger Zeit eine Fixierung, welche nachfolgend aktiv durch die oben genannten chemischen oder physikalischen Einflüsse oder passiv durch Abwarten abgebaut werden.

Die Fixierungsmittel können mechanische oder elektrische Stellelemente, wie Hebel bzw. piezo-elektrische oder andere Aktuatoren umfassen. Dabei ist vorteilhafterweise darauf zu achten, dass die entsprechenden Antriebe oder Fixierungsmittel selber eine Rückmeldung über den auf die Wandungen der Hohlräume ausgeübten Druck oder den Fixierungsgrad abgeben, um Beschädigungen bzw. Verletzungen zu vermeiden. Vorteilhafterweise erfolgt diese Rückmeldung im Falle der Antriebe über die Betrachtung der von den Antrieben aufgenommenen Energie- oder Kraft- oder Impuls- oder Spannungs-/Strom-Zeitverläufe.
Dies hat den Vorteil, dass keine weiteren Sensoren notwendig sind, um den Druck vor Ort, also im Hohlraum direkt zu erfassen.

Die erfindungsgemäße Anordnung kann für jedes menschliche oder tierische Gewebe bzw. Organ eingesetzt werden, vor allem im Rahmen der minimal-invasiven Chirurgie, wo es keine direkte Sichtkontrolle gibt und wo sichergestellt werden muss, dass ein Gewebe bzw. Organ für eine Manipulation an eine spezielle Anordnung, z.B. an eine Vorrichtung zum Punktieren, angeheftet ist.
Die erfindungsgemäße Anordnung kann selbstverständlich auch selber als Instrument zur Durchführung von Manipulationen an den Wandungen eines Hohlraumes oder der dahinter gelegenen Bereiche genutzt werden.

### Beispiele

Ausführungsbeispiele sind in den Zeichnungen 1 bis 13 dargestellt:
Fig.1a zeigt die erfindungsgemäße Anordnung in einem ersten Ausführungsbeispiel in einem Schnitt.
Fig.1b zeigt die Anordnung aus Fig. 1a ebenfalls im Schnitt bei der Bewegung nach rechts.
Fig.2a zeigt eine Schnittdarstellung einer weiteren vorteilhaften Ausführungsform.
Fig.2b zeigt die Anordnung aus Fig. 2a ebenfalls im Schnitt bei der Bewegung nach rechts.
Fig. 3a zeigt eine Schnittdarstellung einer weitern besonders vorteilhaften Ausführungsform.
Fig. 3b zeigt die Anordnung aus Fig. 3b in der Bewegung nach rechts.
Fig. 4 zeigt eine Schnittdarstellung einer besonders schlank gestalteten Ausführungsform.
Fig. 5 zeigt eine Schnittdarstellung einer rotationssymmetrischen Ausführungsform der erfindungsgemäßen Anordnung.
Fig. 6a und b zeigen eine Schnittdarstellung einer ganz besonders vorteilhaften Ausführungsform der erfindungsgemäßen Anordnung.
Fig. 7-13 zeigen Zeichnungen zu einem Ausführungsbeispiel, das die kontrollierte Führung von Instrumenten vorzugsweise im Herzbeutel des menschlichen oder tierischen Körpers ermöglicht.

Die Abbildungen werden im Folgenden näher beschrieben:

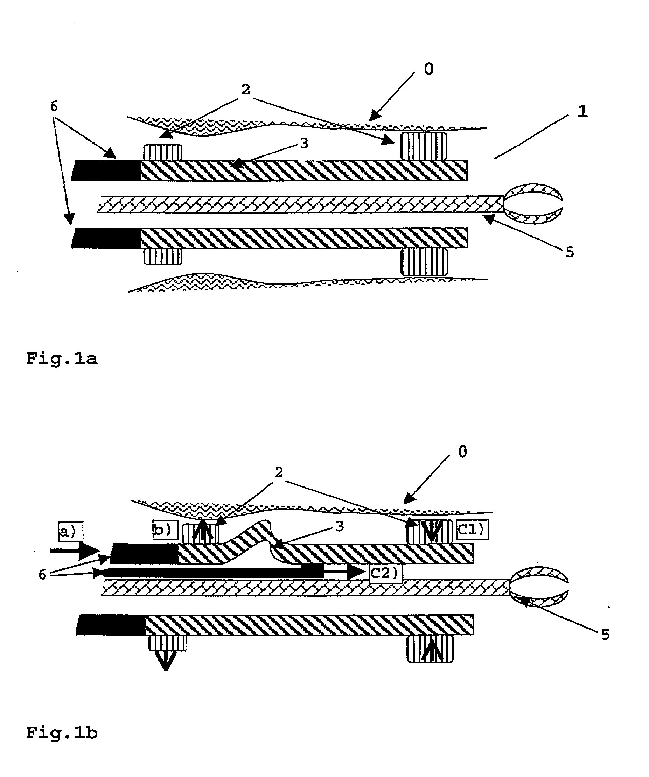

Fig. 1 zeigt die Wandungen (0) eines Hohlraumes, sowie die darin eingeführte Anordnung (1), welche ein Instrument (5) führt. Das Instrument kann reibschlüssig oder wiederholt fixier- und/oder lösbar mit der Anordnung verbunden sein. Die Anordnung weist in dieser dargestellten gestreckten Ausführungsform äußere Wandungen (3) auf, welche das Instrument mindestens zum Teil direkt oder indirekt durch nicht dargestellte Abstandselemente führen. Den Wandungen (3) zugeordnet sind Fixierungsmittel (2). An die Wandung (3) nach hinten, d.h. nach links in Fig. 1a sich erstreckend, sind zugeordnete äußere Fortbewegungsmittel (6) dargestellt. Diese können z.B. durch starre einzelne Stangen oder eine starre Wandung ausgebildet sein. Die Fortbewegungsmittel können auch aus anderen Materialien bestehen, sofern diese bei Schub von rechts nach links oder umgekehrt für eine zumindest teilweise Fortbewegung der Anordnung oder eines Teiles der Anordnung, infolge des ausserhalb der Anordnung angelegten Schubes sorgen.

In Fig. 1a sind die Fixierungsmittel im vorderen, rechten Bereich der Anordnung in Form einer Ausdehnung über die Oberfläche der Wandung (3) hinweg aktiviert. Die Ausdehnung oder Erstreckung über die Oberfläche der Wandung hinweg, ist soweit ausgeführt, dass eine Klemmung der Anordnung im vorderen Teil mit der Wandung des Hohlraumes erfolgt. Die entsprechenden Versorgungsleitungen oder Ausnehmungen oder Kanäle für den Betrieb Fixierungsmittel etwa in Form von elektrischen Leitungen oder Ausnehmungen für elektrische Antriebe oder Kanäle für die Zu- und Abfuhr von Gasen oder Flüssigkeiten unter Druck sind weder hier noch in den folgenden Figuren dargestellt.

Die Fig. 1b zeigt die Situation, ausgehend von der in Fig. 1a, nach Anwendung von Schub auf das obere Fortbewegungsmittel (6). Das Material der Wandung (3) der Anordnung ist so elastisch oder plastisch ausgebildet, dass es bei Überschreitung einer bestimmten Schubkraft zu einer Verformung und infolge dessen zu einer Verkürzung oder Stauchung der Wandung (3) der Anordnung kommt.

Nach dieser Verkürzung werden, wie in Fig. 1b durch die Buchstaben dargestellt, nachfolgend b) die beiden linken Fixierungsmittel (2) in der Form aktiviert, dass eine Klemmung der Anordnung im entsprechenden Bereich des Hohlraumes resultiert, anschließend c1) die beiden rechten Fixierungsmittel in der Form aktiviert, dass die in Fig. 1a dargestellte Klemmung im rechten Bereich aufgehoben wird. Je nach Beschaffenheit des Materials der Wandung (3) erfolgt danach eine Streckung und damit Verlängerung der oberen Wandung auf den Längenwert vor Ausübung des Schubes.

Ist das Material der Wandung lediglich plastisch, so ist durch Betätigung des Fortbewegungsmittels 6, welche in Fig. 1b an dem Instrument anliegend dargestellt ist, die Streckung im Schritt c2) herbeizuführen.

Durch Schritt c1) oder/und c2) resultiert eine Versetzung der Wandung nach rechts und damit eine Fortbewegung der Anordnung unter gleichzeitiger Führung des Instruments (5).

Falls keine Verkürzung und Streckung im unteren Bereich der Wandung herbeigeführt wird, kann nach den Schritten c1) oder/und c2) eine Verkippung beobachtet werden.

Diese Verkippung innerhalb des Hohlraumes kann ebenso durch verschieden starke Aktivierung der Fixierungselemente im Sinne einer Erstreckung über die Oberfläche der Wandung erzeugt werden oder verstärkt werden.

Im Falle einer elastischen oder plastischen Wandung (3) ist dadurch auch eine Abknickung der Anordnung erzielbar.

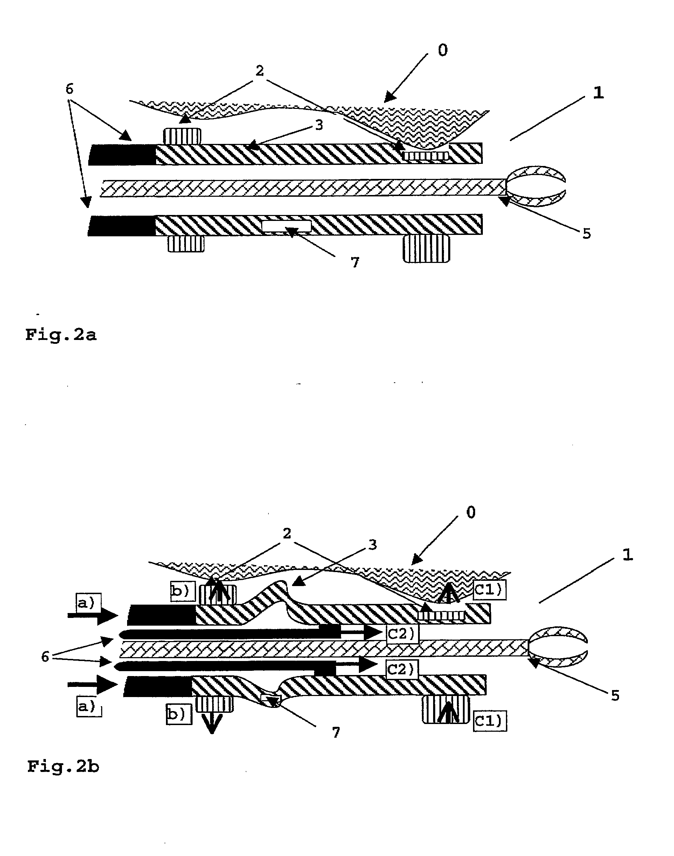

Fig. 2 zeigt eine vorteilhafte Ausführungsform der erfindungsgemäßen Anordnung mit zumindest in ihrer Länge oder auch in ihrer Breite (nicht dargestellt) variabel ausgebildeten Wandungen. Dabei werden, wie in Fig. 2a und 2b dargestellt, Mittel zur Erzeugung eines Unterdrucks als Fixierungsmittel (2) im rechten Bereich der Anordnung eingesetzt. Als Unterdruck erzeugende Mittel können auch Saugnäpfe oder allgemein alle Mittel betrachtet werden, welche durch Anwendung eines Unterdruckes oder einer Druckminderung eine Fixierung der Anordnung an zumindest einem Teil der Wandung des Hohlraumes erzeugen.

Fig. 2a zeigt wie eine Fixierung der Anordnung im rechten (vorderen) Bereich durch Einsaugung eines Teiles der Wandung erzeugt wurde. Darüber hinaus ist in Fig. 2a ein Volumenreduktionselement (7) innerhalb der unteren Wandung der Anordnung dargestellt, womit diese Wandung in ihrer Länge variabel ausgebildet ist.

In Fig. 2b ist für die obere Wandung der gleiche Vorgang wie in Fig. 1b dargestellt, jedoch erfolgte in diesem Ausführungsbeispiel hier die Fixierung durch ein Unterdruck erzeugendes Fixierungsmittel. Deutlich zu erkennen ist wiederum die Verkürzung der Wandung, d.h. die Stauchung der Wandung, welche nach Ausführung der Schritte b) und c1) oder c2) wieder aufgehoben wird.

Der untere Teil der Wandung (3) erfährt keine so große Auslenkung, wie der obere Teil, da das Volumenreduktionselement (7), welches z.B. als durch Unterdruckerzeugung im Volumen verkleinerbare elastische Zelle oder als Piezoelement ausgeführt sein kann, für eine Verkürzung des unteren Teiles der Wandung (3) sorgt.

Auch hier erfolgt -wie in Fig. 1a und 1b- durch alle Varianten c1 oder/und c2 eine Versetzung der Anordnung nach rechts.

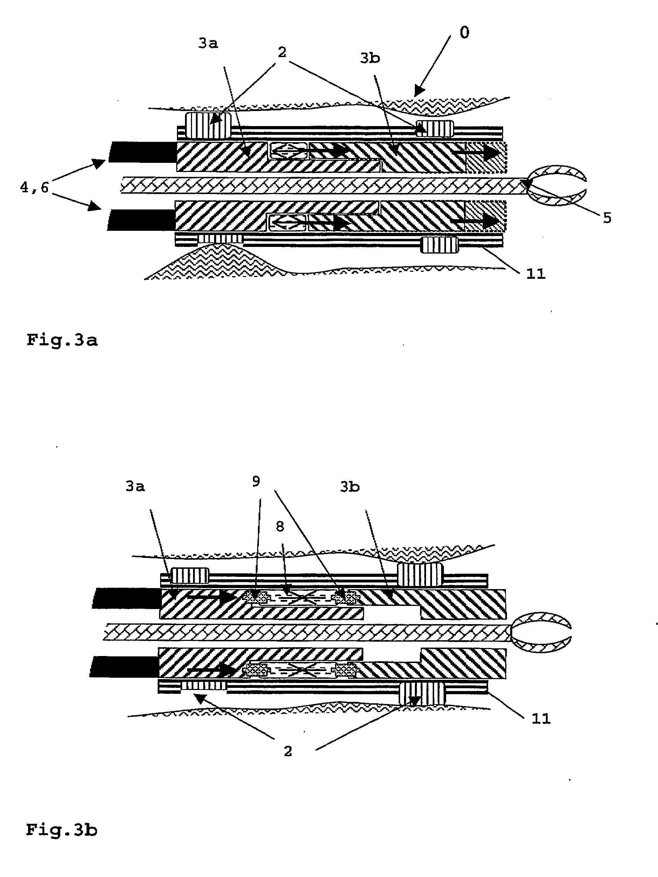

Fig. 3 zeigt eine weitere vorteilhafte Ausführungsform der erfindungsgemäßen Anordnung, bei welcher die Wandung zwischen den mindestens zwei beabstandet voneinander angeordneten Fixierungsmitteln aus mindestens zwei gegeneinander verschieblichen Teilen (3a, 3b) ausgeführt ist.

Fig. 3a zeigt die Anordnung nach Betätigung der linken beiden Fixierungsmittel in der Form, dass eine Fixierung der Anordnung im linken Bereich resultiert. Nach dieser Fixierung, erfolgt dann eine Aktivierung der inneren Fortbewegungsmittel, in der Form, dass sich diese so ausdehnen, dass die rechten Teile (3b) der Wandung (3) nach rechts geschoben werden (durch Pfeile im Fettdruck dargestellt).

Fig. 3b zeigt dann die Anordnung nach Lösung der beiden linken Fixierungsmittel und nach Aktivierung der Fixierung im rechten Bereich der Anordnung.
Zur Fortbewegung der Anordnung (1) nach rechts, unter Führung des Instrumentes (5) nach rechts werden -wie in Fig. 3b durch die Pfeile im Fettdruck dargestellt- innere Fortbewegungsmittel (8) aktiviert, welche den linken Teil (3a) der Wandung (3) nach rechts, hin zum fixierten rechten Bereich der Wandung ziehen.
Die inneren Fortbewegungsmittel (8) können dabei in Form von elastischen, im Volumen oder/und in der Ausdehnung unter anderem parallel zum Instrument, d.h. im wesentlichen in einer Raumrichtung reduzierbaren Elementen, wie Schläuchen oder in Form von Piezoelementen etc. ausgeführt sein.

Im Falle, dass nur eine Ausdehnung oder Kontraktion möglich ist, ist den inneren Fortbewegungsmitteln (8) eine Feder oder ein anderes Vorspannungselement (9) zugeordnet. In Fig. 3b wird die Kraftwirkung von den inneren Fortbewegungsmitteln (8) auf die Teile (3a, b) durch entsprechende Kraftübertragungselemente (9) übertragen. Diese sind aber nicht zwingend erforderlich, sofern eine andere Fixierung zwischen den Mitteln (8) und den Teilen (3a, b) die typischerweise auftretenden Kräfte übertragen kann.

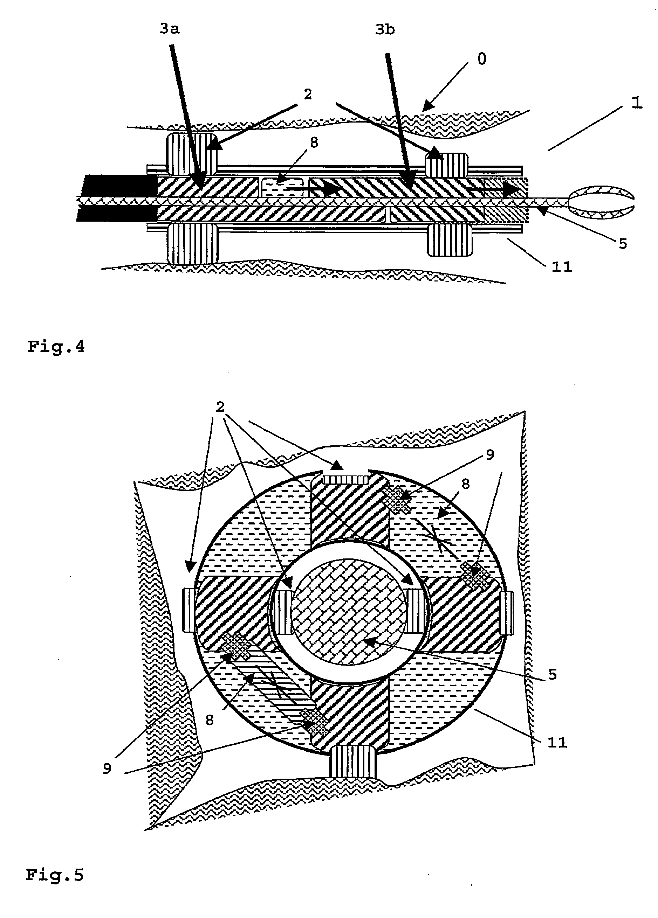

Fig. 4 zeigt eine besonders schlanke, vorteilhafte Ausführungsform der Anordnung (1). Dabei ist das Instrument zwischen den beiden gegeneinander verschieblichen Teile (3a,b) der Anordnung angeordnet. In diesem Ausführungsbeispiel reicht die Vorsehung von einem inneren Fortbewegungsmittel (8). Wie in den Figuren 3a, 3b ist auch in dem entsprechenden Ausführungsbeispiel der Fig. 3 ausserhalb der Wandungen (3a,b) noch eine Ummantelung (11) vorgesehen, welche zumindest Teilbereiche der mindestens zwei gegeneinander beweglichen Teile (3a,b) der Wandung, relativ zu den Teilen, beweglich abdeckt. Dadurch werden eventuelle Verletzungen der Wandung des Hohlraumes vermindert.

Fig. 5 zeigt eine besonders vorteilhafte Ausführungsform für die umfängliche Fortbewegung. Die verschiedenen Bauelemente der erfindungsgemäßen Anordnung (1) sind mit den gleichen Ziffern oder Buchstaben bezeichnet, wie in den vorstehenden Figuren.

Über den Umfang der Wandung der Anordnung (1) verteilt, weist die Anordnung wiederum von einander in Fortbewegungsrichtung beabstandete Fixierungsmittel (2) auf. Wie in Fig. 5 unten dargestellt, ist das untere Mittel aktiviert und daher mit der Wandung des Hohlraumes fixiert. Durch Aktivierung der inneren Fortbewegungsmittel (8) (im linken unteren Quadranten der Fig.) kann dann eine Kontraktion (nicht dargestellt) bewirkt werden. Durch Aktivierung des linken Fixierungsmittels (2) und nachfolgende Lösung des unteren Fixierungsmittels kann dann nach den gleichen Prinzipien, wie in den vorangegangenen Figuren eine umfängliche Bewegung der Anordnung gegen den Uhrzeigersinn bewirkt werden.

Für den Fall, dass das Instrument(5, in der Mitte der Fig. 5 im Schnitt dargestellt) entsprechend mit rotiert werden soll, ist zwischen den Wandungen der Anordnung und dem Instrument mindestens ein weiteres Fixierungsmittel (2) vorgesehen, welches das Instrument am entsprechenden Wandungsteil fixiert und mit der umfänglichen Bewegung der Anordnung mit dreht.

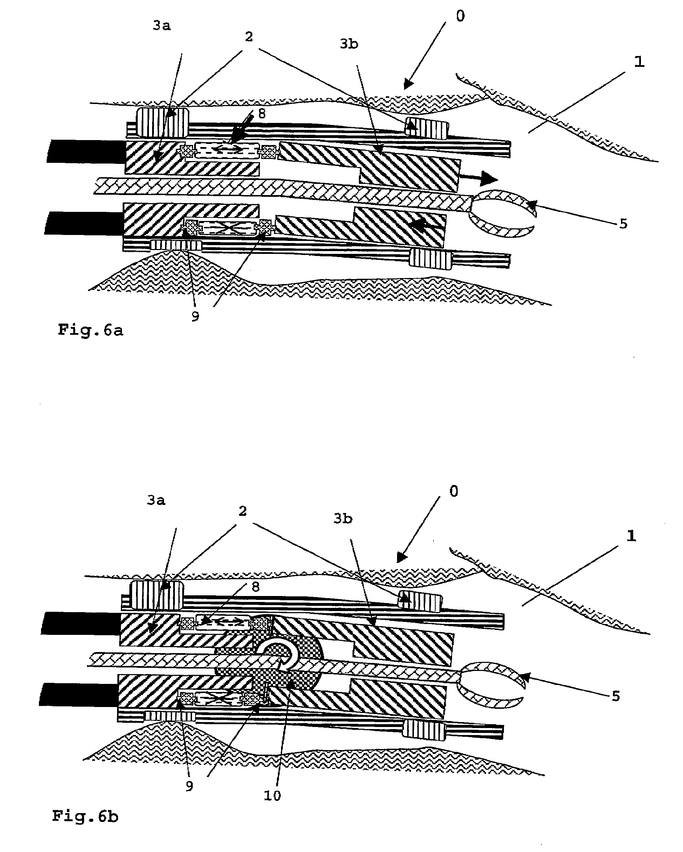

Fig. 6a,b zeigt eine weitere ganz besonders vorteilhafte Ausführungsform der Anordnung. Darin ist die Anordnung aktiv, d.h. durch die inneren Fortbewegungsmittel abknickbar, um auch in abgewinkelten Hohlräumen eine Führung eines Instrumentes und die Fortbewegung der Anordnung zuverlässig zu gewährleisten.

Fig. 6a zeigt zu dieser Ausführungsform eine Variante, bei der die inneren Fortbewegungsmittel (8) getrennt von einander und auch im Ausmaß getrennt von einander zur Vergrößerung oder Verringerung der Ausdehnung in mindestens einer Raumrichtung aktivierbar ausgeführt sind. Die Ummantelung (11) ist für diese Ausführungsform elastisch oder plastisch ausgeführt. Um Rückstellungskräfte nicht durch die prinzipiell dafür geeigneten inneren Fortbewegungsmittel aufbringen zu müssen, ist es für die entsprechenden Fälle vorgesehen, die Ummantelung aus einem Gewebe ausgeführt zu wählen, welches eine Rückstellkraft aufweist.

Fig. 6b zeigt dazu eine zweite Variante zu dieser Ausführungsform, bei der die Anordnung (1) zusätzlich Abwinkelungsmittel (10) in Form einer drehbar gelagerten Scheibe aufweist. Natürlich können dafür auch alle möglichen Varianten von Gelenken oder Getrieben eingesetzt werden, welche eine Abwinkelung der mindestens zwei Teile der Wandung (3a,b) bewirken.

Nicht dargestellt ist eine weitere Variante zu dieser Ausführungsform, in welcher die Inneren Fortbewegungsmittel (8) nicht mehr vorgesehen sind, da das Abwinkelungsmittel aktiv, d.h. mit einem eigenen Antrieb versehen ausgeführt ist.

Insbesondere mit der letzten Ausführungsform ist es deutlich einfacher möglich, ein Instrument auch um eine Ecke herum zuverlässig zu führen und eine einmal angesteuerte Position relativ zum Hohlraum wieder zuverlässig anzusteuern, da die Führung des Instrumentes nur noch translatorische oder drehende Bewegungen des Instrumentes zulässt.

Eine weitere vorteilhafte -nicht dargestellte- Ausführungsform, sieht den Einsatz von Sensoren zur Überwachung der Fixierung, etwa in Form von Drucküberwachungs- oder im Falle von elektrischen Fixierungsmitteln, Strom-/Spannungs- und oder Energie-/Leistungsüberwachungsmitteln, sowie die entsprechenden visuellen, taktilen oder die anderen menschlichen Sinne anregenden Anzeigevorrichtungen vor.

Nach Kalibrierung der Anordnung etwa für ausgewählte Hohlräume im menschlichen Körper, sowie der entsprechenden Fixierungs-Aktivator- und -Überwachungsmittel ist es möglich die Anordnung mit dem Instrument automatisiert bis zu einer gewissen Stelle, etwa im Körper eines Menschen -nach dem Einsetzen in den jeweiligen Hohlraum- fortzubewegen. Dies wird insbesondere in abgewinkelten Hohlräumen durch die Ausführungsbeispiel gemäß Fig. 6a und 6b und insbesondere in Kombination dieses Ausführungsbeispieles mit dem gemäß Fig. 5 ermöglicht.

Der Anordnung sind dazu entsprechende Speicher und Steuer-/Regelsignalgeber etwa in Form eines PCs zu zuordnen.

Es ist dem Fachmann unmittelbar ersichtlich, dass die erfingungsgemäße Anordnung (1) durch Verwendung eines abwinkelbaren Instrumentes zumindest, sofern die Wandungen der Anordnungen flexibel ausgeführt sind, auch abwinkelbar ist.

Ein weiteres Ausführungsbeispiel, sieht vor, dass die Anordnung so ausgeführt ist, dass ein Teil der Fixierungsmittel am zu führenden Instrument oder auch an einer weiteren, in den bisher erläuterten Figuren nicht gesondert dargestellten inneren Wandung (4) der Anordnung angeordnet sind. Im Falle der Vorsehung von Fixierungsmitteln an der inneren Wandung (4) der Anordnung, ist diese relativ verschiebbar zur äußeren Wandung (3a, 3b) ausgeführt und weist entweder innere Fortbewegungsmittel (8), wie z.B. in Fig. 1, 3a und 3b gezeigt auf oder ist mit äußeren Fortbewegungsmitteln (6), wie z.B. einem Stab oder einem stauch- und zugfesten Draht gekoppelt. Diese Ausführungsform ist besonders bevorzugt zur kontrollierten Bewegung von Instrumenten vorzugsweise im Herzbeutel des menschlichen oder tierischen Körpers verwendbar. Dabei erfolgt die Führung des Instrumentes vorzugsweise über eine zuverlässig erkannte und signalisierte Fixierung der äußeren Wandung (3a, 3b) oder der inneren Wandung (4), welche dazu aus der inneren Wandung herausragt, an ein umliegendes Gewebe. Beispielsweise ermöglicht diese Ausführungsform auch die Anbringung von Stimulationselektroden an einem definierten Ort auf der Herzoberfläche, der abgewinkelt zum Punkt des einführenden Instrumentes liegt. Verfügbare Instrumente sind für diese Anwendung nicht geeignet, wie das Beispiel eines steuerbaren Endoskops verdeutlicht. Nach Abwinkelung des Endoskopkopfes erfordert die nachfolgende Bewegung in Richtung der Abwinkelung eine nicht verschiebbare und den Endoskopkopf abweisende führende Wand. Wenn diese Wand nicht vorhanden ist, erfolgt trotz Abwinkelung des Endoskopkopfes die Bewegung nur in Verlängerung des Punktes, von dem aus das Endoskop vorgeschoben wird. Der abgewinkelt liegende Zielort wird daher nicht erreicht. Da im Beispiel der Implantation einer Elektrode auf der Herzoberfläche eine erforderliche abweisende Wand für das Endoskop nicht vorhanden ist, fehlt die Führung und es wird der für die Elektrodenanbringung geeignete Ort nicht erreicht. Auch kann der gewünschte Ort nicht allein über eine Abwinkelung des Kopfteiles eines Endoskops erreicht werden, da der Kopfteil zu kurz ist. Ein längeres Kopfteil würde wegen der örtlichen Enge keine Abwinkelung ermöglichen und Verletzungen wären nicht auszuschließen.

Um die bestehenden Probleme zu lösen, wird in diesem Ausführungsbeispiel die Anordnung so ausgewählt, dass ein Teil der Fixierungsmittel (2,2a) an der äußeren Wandung (3a,3b) der Anordnung und ein Teil der Fixierungsmittel an einer inneren, gegen die äußere Wandung (3a,b) verschieblichen Wandung (4) oder am zu führenden Instrument (5) selber angeordnet ist. Die beiden Wandungen oder die äußere Wandung und das Instrument lassen sich dann entweder durch den Einsatz von äußeren Fortbewegungsmitteln (6) (z.B. einen stauch- und/oder zugfesten Draht oder Schlauch) oder in Kombination mit inneren Fortbewegungsmitteln (8) zeitlich und räumlich versetzt fortbewegen. Eine für den oben angegebenen Fall des Eintritts in Herzgefäße erforderliche Abwinkelung erfolgt nach einer Fixierung der äußeren Wandung (3a,b) auf der Herzoberfläche oder auf dem Perikard. Diese Ausführungsform kann für jedes menschliche oder tierische Gewebe bzw. Organ eingesetzt werden, vor allem im Rahmen der minimal-invasiven Chirurgie, wo es keine direkte Sichtkontrolle gibt und wo ein definierter Ort aus den genannten Gründen nicht über ein konventionelles steuerbares Endoskop erreicht werden kann.

In einer Abwandlung dieser Ausführungsform ist vorgesehen, die äußere Wandung (3a,3b) zweigeteilt auszuführen, wobei der sich dann ergebende Kopf- und Hauptteil mit einem Abwinkelungsmittel verbunden ausgeführt ist. Die Abwinkelung des Kopfteils erfolgt z. B. über einen Seilzug. Die für die gezielte Abwinkelung des Kopfteils notwendige Fixierung des Hauptteils wird z.B. über eine mechanische Feststellung oder durch Erzeugung eines Unterdruckes erreicht. Eine der Anordnung zugeordnete Fixierungserfassungsvorrichtung kann ein oder mehrere Erfassungsmittel aufweisen, u.a. druckabhängige, akustische sowie optische. Mittels einer Anzeigevorrichtung werden Signale, die von der Fixierungserfassungsvorrichtung erfasst werden, in Anzeigesignale umgewandelt.

Ausführungsbeispiele und Darstellungen zur Anatomie des Herzens sind in den Zeichnungen 7 bis 12 dargestellt.

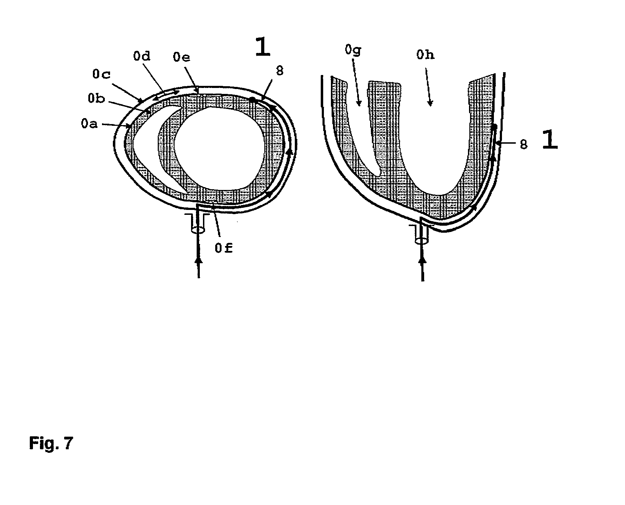

Fig. 7 zeigt das Herz im Querschnitt (links) und Längsschnitt (rechts).

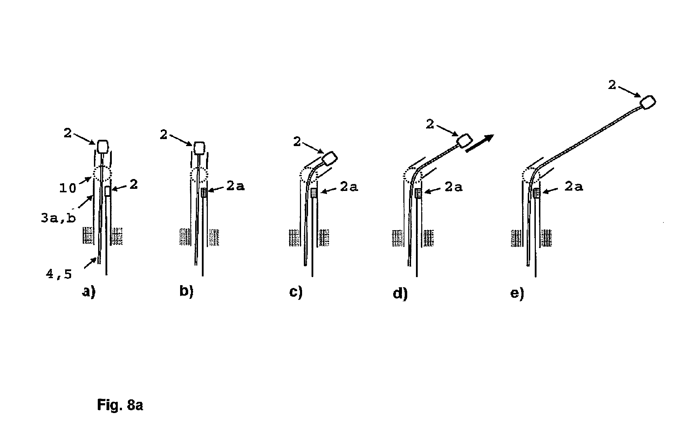

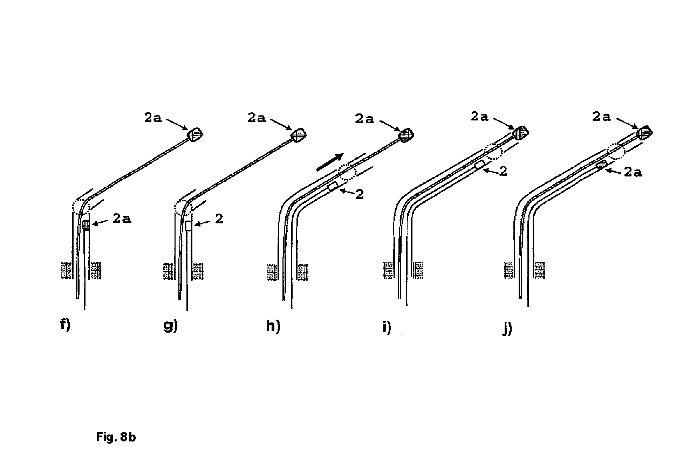

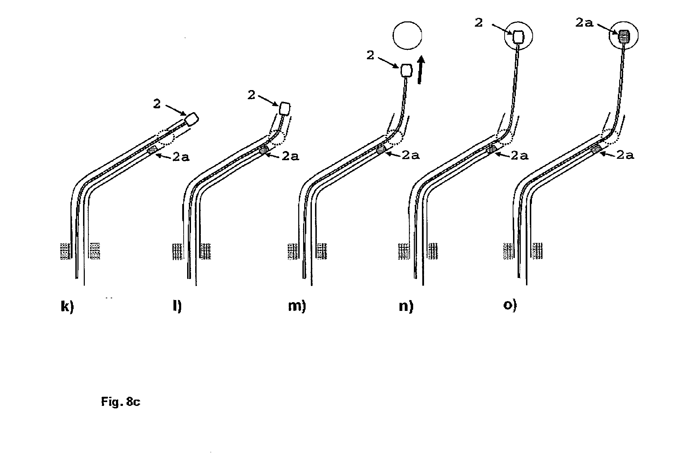

Fig. 8 zeigt schematisch Schritte der zeitlich und räumlich versetzten Bewegung der äußeren Wandung (3a,b) und der inneren Wandung (4) oder des Instrumentes (5) an welchem ein Teil der Fixierungsmittel (2,2a) angeordnet ist.

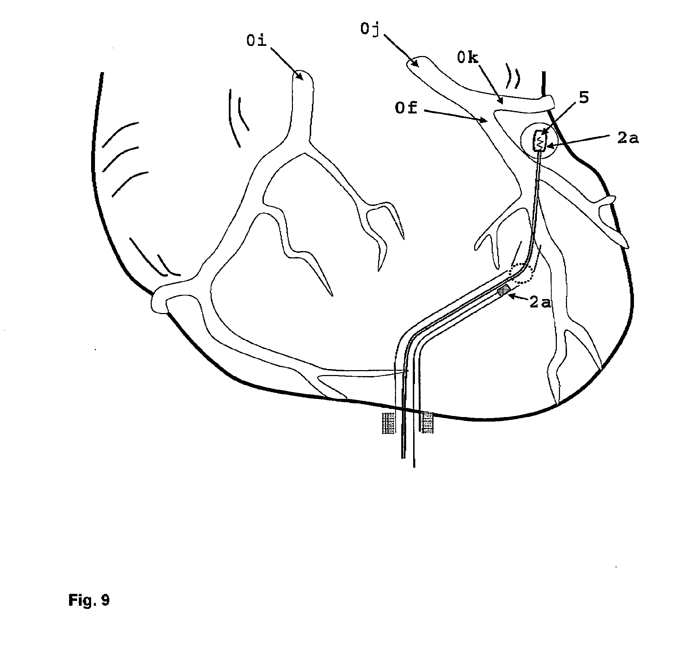

Fig. 9 zeigt den Zustand, der in der Fig. 8c rechts dargestellt ist, projiziert auf die Herzoberfläche.

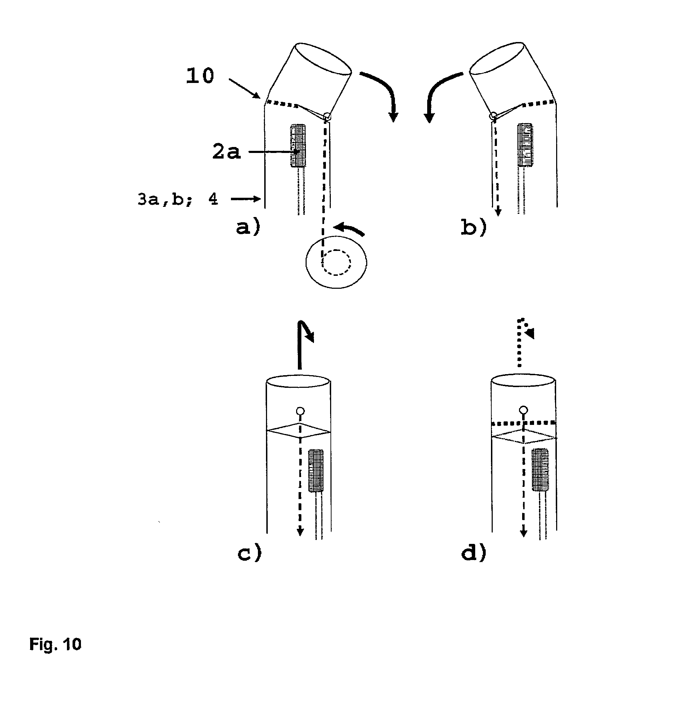

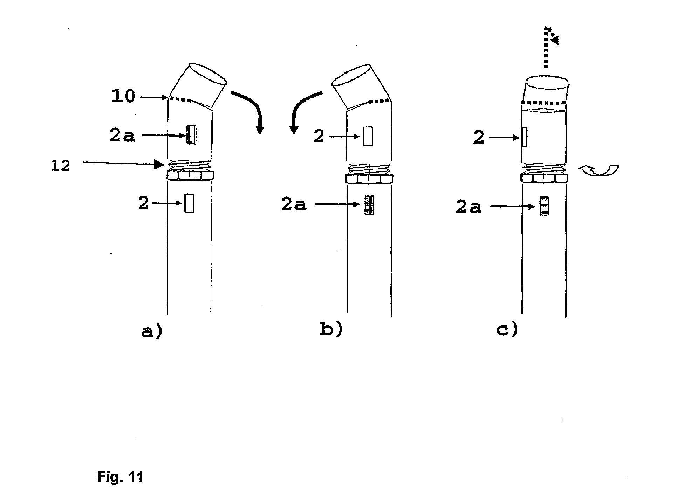

Fig. 10 zeigt mehrere über Ablenkungsmittel bewegbare Kopfteile der äußeren Wandung (3a,b), die jeweils ein Fixierungsmittel im Haupteil aufweisen und -nicht dargestellt- auch im Kopfteil aufweisen können. Fig. 11 zeigt eine Ausführungsform der erfindungsgemäßen Anordnung, wobei die äußere Wandung (3a,b) dreigeteilt ausgeführt ist und in den unteren beiden Teilen, welche durch Drehmittel (hier in Form eines Gewindes) von einander getrennt sind, jeweils ein Fixierungsmittel aufweisen. Die Verbindung zwischen den oberen beiden Teilen wird durch ein Ablenkungsmittel (10) hergestellt. In diesem besonders vorteilhaften Ausführungsbeispiel ist das Kopfteil gegenüber dem Hauptteil somit drehbar ausgeführt.

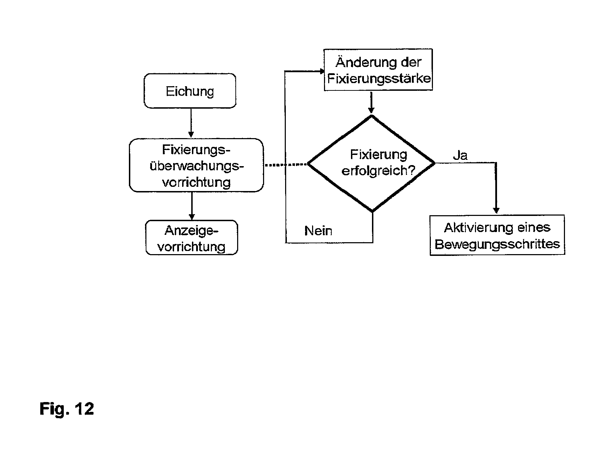

Fig. 12 zeigt das Verfahren und Steuerungsmittel bei der Überwachung eines aktivierten Fixierungsmittels.

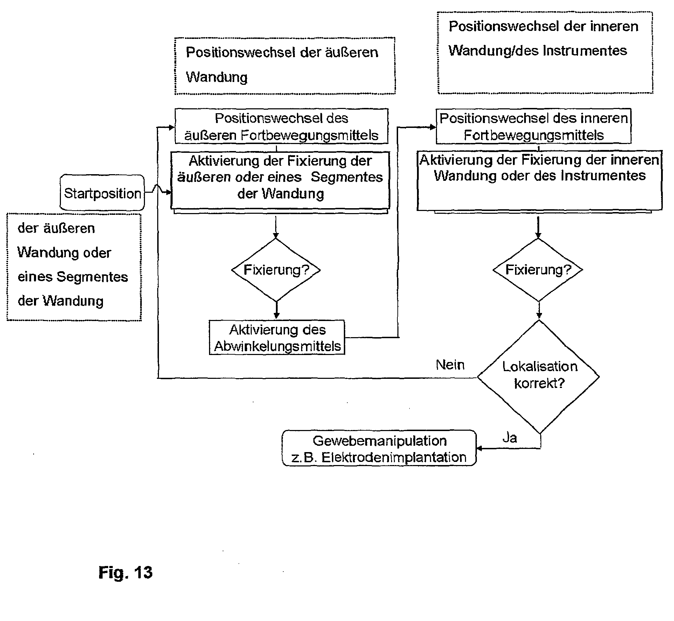

Fig. 13 zeigt beispielhaft Verfahren und Steuerungsmittel bei der Bewegung der äußeren Wandung und der inneren Wandung oder des Instrumentes.

Die Abbildungen werden im Folgenden näher beschrieben:
Fig. 7 Dargestellt sind das Epikard (0a), das der Oberfläche des Myokards (0b) aufliegt und das parietale Perikard (0c) und der Hohlraum des Herzbeutels (0d). Eingezeichnet ist das Herzkranzgefäß Ramus interventricularis posterior (0e), das aus der Arteria coronaria dextra und das Herzkranzgefäß Ramus interventricularis anterior (Of), das aus der Arteria coronaria sinistra hervorgeht. Das Herz besteht aus der rechten (0g) und der linken (0h) Herzkammer. Durch einen Zugang (unten) wird ein Instrument, das sich in der Anordnung befindet an einen vorgegebenen Zielort geführt.
Fig. 8a-c zeigt schematisch Schritte der zeitlich und räumlich versetzten Bewegung der äußeren Wandung (3a, 3b) und der inneren Wandung (4) oder des Instrumentes (5) vorzugsweise im Herzbeutel. In Teilfig. a) der Fig. 8a wird die Anordnung in den Herzbeutel eingeführt. Die beiden Fixierungsmittel (2) sind nicht aktiviert. In Teilfig. b) erfolgt eine Aktivierung des Fixierungsmittels (2a) an der äußeren Wandung (3a,b). In c) erfolgt bei erhaltener Fixierung (2a) mit Hilfe eines Abwinkelungsmittels eine Abwinkelung nach rechts. Im Ausführungsbeispiel einer flexiblen Röhre kann z.B. das Kopfteil in Abhängigkeit des gewünschten Ablenkwinkels eine unterschiedlich große Kerbe aufweisen. Das Kopfteil wird z.B. über einen in der Vorrichtung liegenden Seilzug abgewinkelt. In d) und e) erfolgt bei erhaltener Fixierung (2a) der äußeren Wandung die Bewegung der inneren Wandung oder des geführten Instrumentes in Richtung der Abwinkelung. In f) wird auch die innere Wandung oder das geführte Instrument fixiert (2a). In g) wird die Fixierung (2) der äußeren Wandung (3a,b) gelöst. In h) und i) wird die äußere Wandung (3a,b) entlang der fixierten inneren Wandung oder des fixierten Instrumentes nachgeschoben. In j) erfolgt die Fixierung (2a) der äußeren Wandung, dann wird in k) die Fixierung (2) der inneren Wandung oder des Instrumentes gelöst. In l) wird das Kopfteil der äußeren Wandung nach links abgewinkelt und in m) und n) wird die innere Wandung oder das Instrument in Richtung der Abwinkelung vorgeschoben. In o) wird der Zielort erreicht und die innere Wandung (4) oder das Instrument (5) kann fixiert werden. Diese Schritte können beliebig wiederholt werden, wobei die Richtung der Abwinkelung veränderbar ist.
Fig. 9 zeigt den in der Fig. 8c rechts dargestellten Zustand der Anordnung projiziert auf die Herzoberfläche. Eingezeichnet sind Arteria coronaria dextra (0i), Arteria coronaria sinistra (0j), Ramus circumflexus (0k), Ramus interventricularis anterior (0f). Mit dem in der äußeren Wandung (3a,b) oder in der inneren Wandung (4) vorgeschobenen Instrument (5) erfolgt eine Gewebemanipulation, z.B. eine epikardiale Elektrodenimplantation, die durch die Korkenzieherartige Anordnung des Instrumentes (5) angedeutet ist. Bei der Implantation ist die dem Instrument nächstgelegene Wandung (3a,b oder 4) fixiert (2a, um die Elektrodenspitze kenntlich zu machen wurde die Schraffur des aktivierten Fixierungsmittels entfernt).
Fig. 10 zeigt vier verschiedene, über Ablenkungsmittel (10) bewegbare, Kopfteile von äußeren oder inneren Wandungen (3a,b oder 4), die jeweils ein Fixierungsmittel besitzen. Die Abwinkelung erfolgt nach Fixierung an einer Wand durch ein Fixierungsmittel. Im gezeigten Ausführungsbeispiel handelt es sich um eine vorzugsweise flexible Röhre, die in Abhängigkeit des gewünschten Ablenkwinkels eine unterschiedlich große Kerbe aufweist. Das Kopfteil wird z.B. über einen in der Vorrichtung liegenden Seilzug abgewinkelt. Im Beispiel a) kann das Gelenk nach rechts, b) nach links, c) nach vorne und d) nach hinten abgewinkelt werden. Das Fixierungsmittel (2a) befindet sich jeweils an der Unterseite und stellt im Beispiel eine Fixierung auf der Herzoberfläche her. In diesem Ausführungsbeispiel werden vier Anordnungen für die vier angestrebten Abwinkelungen verwendet.
Fig. 11 zeigt ein weiteres Ausführungsbeispiel, wobei die äußere oder innere Wandung (3a,b oder 4) mit zwei Fixierungsmitteln und einem Ablenkungsmittel ausgeführt ist, wobei die vordere Anordnung gegenüber der hinteren Anordnung drehbar ist. Dargestellt ist eine mögliche Kippbewegung nach rechts a), eine mögliche Kippbewegung nach links nach Drehung der gesamten Anordnung um 180° b) und eine mögliche Kippbewegung nach hinten nach Drehung des Kopfteils im Gewinde um 90° im Uhrzeigersinn, wobei während der Drehung die hintere Anordnung fixiert ist c). Die Fixierung (2a) wird bei a), b) und c) jeweils hinten aktiviert. Die beispielhaft gezeigte Anordnung ermöglicht drei Abwinkelungen, wobei nur 2 Fixierungsmittel und ein Gewinde erforderlich sind. Anstelle des Gewindes können auch andere Verbindungen, welche gegeneinander drehbar und gleichzeitig oder von der Drehung losgelöst ausdehnbar sind Anwendung als Dreh- oder und Ausdehnungsmittel (12) finden.
Fig. 12 zeigt beispielhaft das Verfahren und die Steuerungsmittel bei der Überwachung eines aktivierten Fixierungsmittels. Mit Hilfe einer Fixierungsüberwachungsvorrichtung und einer Anzeigeanordnung wird die Fixierung, die eine definierte Bewegung der äußeren oder der inneren Wandung (3a,b oder 4) oder des Instrumentes ermöglicht, kontrolliert.
Fig. 13 Beispielhafte Darstellung des Verfahrens und der Steuerungsmittel bei der Bewegung der äußeren oder/und inneren Wandung, oder des von diesen zumindest teilweise umschlossenen Instrumentes. Falls die gewünschte Lokalisation auf z.B. der Herzoberfläche nicht erreicht ist, wird die Bewegung fortgesetzt. Ist die Lokalisation korrekt, erfolgt z.B. eine Gewebemanipulation. Die Bezeichnung "Fixierung" ist in der Fig. 12 erklärt.

### Bezugszeichenliste

- 0: Wandung eines Hohlraums
- 0a: Epikard
- 0b: Myokard
- 0c: Perikard
- 0d: Herzbeutel
- 0e: Ramus interventricularis posterior
- 0f: Ramus interventricularis posterior
- 0g: Rechte Herzkammer
- 0h: Linke Herzkammer

- 1: Anordnung zur Führung von Instrumenten
- 2, 2a: Fixierungsmittel
- 3, 3a, 3b: Äußere Wandung der Anordnung
- 4: Innere Wandung der Anordnung
- 5: Instrument
- 6: Äußere Fortbewegungsmittel
- 7: Volumenreduktionselemente
- 8: Innere Fortbewegungsmittel
- 9: Vorspannungs- oder Kraftübertragungs-Elemente
- 10: Abwinkelungsmittel
- 11: Ummantelung
- 12: Dreh- oder und Verlängerungsmittel

## Patentansprüche

1. Anordnung (1) zur Führung eines medizinischen Instrumentes (5) innerhalb eines Hohlraumes, umfassend eine Wandung (3, 4) zur zumindest teilweisen Führung des Instrumentes (5) durch einen Abschnitt der Wandung (3, 4), wobei die Anordnung (1) voneinander beabstandete Fixierungsmittel (2,2a) für eine Fixierung der Anordnung relativ zu mindestens einem Teil der Wandung des zu untersuch enden Hohlraumes, aufweist, wobei die äußere oder innere Wandung (3, 4) mehrere Segmente aufweist **dadurch gekennzeichnet, dass**
a) die mehreren Segmente durch innere Fortbewegungsmittel (8) oder/und durch Abwinkelungsmittel (10) oder/und durch Drehmittel (12) oder/und durch Volumenreduktionselemente (7) gegeneinander abknickbar miteinander verbunden ausgeführt sind, sowie in der Länge der einzelnen Segmente oder in der Länge mehrerer verbundener Segmente veränderbar ausgeführt sind.

2. Anordnung nach Anspruch 1 , **dadurch gekennzeichnet, dass** die voneinander beabstandeten Fixierungsmittel (2,2a) getrennt voneinander in der Art aktivierbar ausgeführt sind, dass jedes Fixierungsmittel (2,2a) unabhängig vom anderen zur Fixierung oder/und Lösung des entsprechenden Teiles der Anordnung (1) an der Wandung (0) des Hohlraumes ausgeführt ist.

3. Anordnung nach Anspruch 1 bis 2, **dadurch gekennzeichnet, dass** die Anordnung (1) so ausgeführt ist, dass die Anordnung (1) mindestens eine innere Wandung (4) aufweist, welche gegen die äußere Wandung (3) verschieblich und/oder drehbar ausgeführt ist.

4. Anordnung nach Anspruch 1 bis 2, **dadurch gekennzeichnet, dass** die Anordnung (1) so ausgeführt ist, dass ein Teil der Fixierungsmittel (2,2a) an dem zu führenden Instrument (5) angeordnet sind.

5. Anordnung nach Anspruch 3 bis 4, **dadurch gekennzeichnet, dass** ein Teil der voneinander beabstandeten Fixierungsmittel (2,2a) an der Wandung (3) oder/und dem zu führenden Instrument (5) oder/und an der mindestens einen inneren Wandung (4) angeordnet ist.

6. Anordnung nach einem der vorangehenden Ansprüche, **dadurch gekennzeichnet, dass** die Fixierungsmittel (2,2a) als Unterdruckmittel oder/und Klemmmittel oder/und zumindest temporär adhäsive Mittel ausgeführt sind.

7. Anordnung nach einem der vorangehenden Ansprüche, **dadurch gekennzeichnet, dass** die Fixierungsmittel (2,2a) Sensoren zur Überwachung des Grades der Fixierung aufweisen.

8. Anordnung nach einem der vorangehenden Ansprüche, **dadurch gekennzeichnet, dass** der Anordnung eine Fixierungs-Anzeigevorrichtung zugeordnet ist.

9. Anordnung nach einem der vorangehenden Ansprüche, **dadurch gekennzeichnet, dass** die Anordnung zur Führung mehrerer Instrumente innerhalb der Wandung (3a, b oder 4) ausgeführt ist.

10. Anordnung nach Anspruch 8, **dadurch gekennzeichnet, dass** die Anzeigevorrichtung als Anzeigemittel Licht-, Schall, Geruchs oder taktile Signale abgebende Mittel aufweist.

11. Anordnung nach wenigstens einem der vorangehenden Ansprüche, **dadurch gekennzeichnet, dass** die Anordnung (1) wenigstens ein äußeres Fortbewegungsmittel (6) aufweist.

12. Anordnung nach einem der vorangehenden Ansprüche, **dadurch gekennzeichnet, dass** die Anordnung (1) eine Ummantelung (11) aufweist.

13. Medizinische Vorrichtung oder Vorrichtung zur Untersuchung von Hohlräumen, umfassend eine Anordnung nach einem der Ansprüche 1 bis 12.

## Claims

1. Assembly (1) for guiding a medical instrument (5) within a cavity, comprising a wall (3, 4) for guiding at least a part of the instrument (5) through a portion of the wall (3, 4), the assembly comprising fixation means (2, 2a) which are at a distance from one another for fixing the assembly in position relative to at least one part of the wall of the cavity to be examined, the outer or inner wall (3, 4) comprising a plurality of segments, **characterised in that**
a) the plurality of segments are designed to be interconnected such as to be bendable with respect to one another by inner transportation means (8) and/or by bending means (10) and/or by rotation means (12) and/or by volume reduction elements (7), and are designed so that the individual segments or the plurality of connected segments are variable in length.

2. Assembly according to claim 1, **characterised in that** the fixation means (2, 2a) at a distance from one another are designed in such a way that they can be activated separately from one another such that each fixation means (2, 2a) is designed to be independent from the other for the fixation or/and detachment of the corresponding part of the assembly (1) to and/or from the wall (0) of the cavity.

3. Assembly according to claims 1 and 2, **characterised in that** the assembly (1) is designed in such a way that the assembly (1) comprises at least one inner wall (4), which is designed to be movable and/or rotatable against the outer wall (3).

4. Assembly according to claims 1 and 2, **characterised in that** the assembly (1) is designed in such a way that a part of the fixation means (2, 2a) is arranged on the instrument (5) to be guided.

5. Assembly according to claims 3 and 4, **characterised in that** a part of the fixation means (2, 2a) which are at a distance from one another is arranged on the wall (3) and/or the instrument (5) to be guided and/or on the at least one inner wall (4).

6. Assembly according to any of the preceding claims, **characterised in that** the fixation means (2, 2a) are designed as vacuum means and/or clamping means and/or at least temporarily adhesive means.

7. Assembly according to any of the preceding claims, **characterised in that** the fixation means (2, 2a) comprise sensors for monitoring the degree of fixation.

8. Assembly according to any of the preceding claims, **characterised in that** a fixation display device is assigned to the assembly.

9. Assembly according to any of the preceding claims, **characterised in that** the assembly is designed for guiding a plurality of instruments within the wall (3a, b or 4).

10. Assembly according to claim 8, **characterised in that** the display device comprises means emitting light, sound, scent or tactile signals as a display means.

11. Assembly according to at least one of the preceding claims, **characterised in that** the assembly (1) comprises at least one outer transportation means (6).

12. Assembly according to any of the preceding claims, **characterised in that** the assembly (1) comprises a casing (11).

13. Medical device or device for examining cavities, comprising an assembly according to any of claims 1 to 12.

## Revendications

1. Dispositif (1) pour guider un instrument médical (5) à l'intérieur d'une cavité, lequel dispositif comprend une paroi (3, 4) pour le guidage au moins partiel de l'instrument (5) dans un tronçon de la paroi (3, 4), le dispositif (1) comportant des moyens de fixation (2, 2a) qui sont distants les uns des autres et qui sont destinés à la fixation du dispositif par rapport à au moins une partie de la paroi de la cavité à examiner, la paroi extérieure ou intérieure (3, 4) comportant plusieurs segments,
**caractérisé en ce que**
a) les segments sont réalisés, assemblés les uns aux autres, de manière à pouvoir être pliés les uns par rapport aux autres par des moyens de déplacement (8) ou/et par des moyens de pliage (10) ou/et par des moyens de rotation (12) ou/et par des moyens de réduction de volume (7) et ils sont aussi réalisés de manière à pouvoir être modifiés quant à la longueur des segments individuels ou à la longueur de plusieurs segments assemblés.

2. Dispositif selon la revendication 1, **caractérisé en ce que** les moyens de fixation (2, 2a) distants les uns des autres sont réalisés de manière à pouvoir être activés de telle sorte séparément les uns des autres que chaque moyen de fixation (2, 2a) est réalisé indépendamment de l'autre pour la fixation et/ou la libération de la partie correspondante du dispositif (1) à/de la paroi (0) de la cavité.

3. Dispositif selon la revendication 1 à 2, **caractérisé en ce que** le dispositif (1) est réalisé de telle sorte que le dispositif (1) comporte au moins une paroi intérieure (4) qui est réalisée de manière à pouvoir être déplacée et/ou tournée par rapport à la paroi extérieure (3).

4. Dispositif selon la revendication 1 à 2, **caractérisé en ce que** le dispositif (1) est réalisé de telle sorte qu'une partie des moyens de fixation (2, 2a) est agencée sur l'instrument (5) à guider.

5. Dispositif selon la revendication 3 à 4, **caractérisé en ce qu'**une partie des moyens de fixation (2, 2a) distants les uns des autres est agencée sur la paroi (3) ou/et sur l'instrument à guider (5) ou/et sur la au moins une paroi intérieure (4).

6. Dispositif selon l'une des revendications précédentes, **caractérisé en ce que** les moyens de fixation (2, 2a) sont réalisés sous la forme de moyens de dépression ou/et de moyens de serrage ou/et de moyens au moins temporairement adhésifs.

7. Dispositif selon l'une des revendications précédentes, **caractérisé en ce que** les moyens de fixation (2, 2a) comportent des capteurs pour surveiller le degré de la fixation.

8. Dispositif selon l'une des revendications précédentes, **caractérisé en ce qu'**un dispositif d'indication de fixation est associé au dispositif.

9. Dispositif selon l'une des revendications précédentes, **caractérisé en ce que** le dispositif est réalisé pour guider plusieurs instruments à l'intérieur de la paroi (3a, b ou 4).

10. Dispositif selon la revendication 8, **caractérisé en ce que** le dispositif d'indication comporte comme moyens d'indication des moyens délivrant des signaux lumineux, sonores, olfactifs ou tactiles.

11. Dispositif selon l'une des revendications précédentes, **caractérisé en ce que** le dispositif (1) comporte au moins un moyen de déplacement extérieur (6).

12. Dispositif selon l'une des revendications précédentes, **caractérisé en ce que** le dispositif (1) comporte une enveloppe (11).

13. Appareil médical ou appareil pour l'examen de cavités, comprenant un dispositif selon l'une des revendications 1 à 12.
